**Europäisches Patentamt**

**European Patent Office**

**Office européen des brevets**

(11) Publication number : **0 488 821 A1**

# (12) EUROPEAN PATENT APPLICATION

(21) Application number : **91311184.5**

(22) Date of filing : **02.12.91**

(51) Int. Cl.⁵ : **A61M 25/02**

(30) Priority : **30.11.90 ZA 909640**

(43) Date of publication of application :
**03.06.92 Bulletin 92/23**

(84) Designated Contracting States :
**AT BE CH DE DK FR GB IT LI NL SE**

(71) Applicant : **OP-CO Medical Products, Limited**
**30 Rockefeller Plaza, Room 5455**
**New York, New York 10112 (US)**

(72) Inventor : **Guastella, Janice Ann**
**150 East 69th Street**
**New York, New York 10021 (US)**

(74) Representative : **Sheard, Andrew Gregory et al**
**Kilburn & Strode 30, John Street**
**London WC1N 2DD (GB)**

(54) **Pliable securing device for medical needles.**

(57)    A protecting and restraining apparatus (10) for securing a medical needle (11) inserted into a patient. The apparatus of the present invention including a pliable material (12) that can be moulded by finger pressure to encompass the inserted medical needle. An adhesive sheet is used to secure the pliable material to the patient.

FIGURE 3

**EP 0 488 821 A1**

## Technical Field

The present invention relates generally to securing medical needles or cannulae to patients by using pliable pads pressed to encompass the needles or cannulae adjacent the akin, and more specifically the pliable pads are putty-like to facilitate moulding about needles or cannulae adjacent the skin with the moulded pliable pad retaining the needle held in place by an adhesive strip.

## Background Art

If a medical needle or cannula is inserted into a patient for draining liquids or gases into or out of a patient over an extended period of time, it needs to be retained in a stable and fixed positional relationship to the patient. Retention of the inserted device with respect to the patient is required in order to minimize patient discomfort, tissue irritation, tissue infiltration, vein puncture, and the risk of infection.

Devices currently used to retain inserted medical devices with respect to patients include tape, tape and gauze, transparent occlusive dressings and other similar devices using foams and occlusive dressings, and clips for securing inserted medical devices with the clips retained by wrist encircling straps. All of these prior devices fail to provide systems that can be affixed to an inserted medical needle or cannula without substantially moving the inserted medical device. After a medical device is inserted into a patient, it can be supported in a position with respect to the patient that is most comfortable for the patient and should be retained by a securing device. Most prior known securing devices unavoidably result in moving inserted medical devices while the securing devices are being installed and usually end up retaining the inserted medical devices in different positions with respect to the patient. These deficiencies are unavoidably associated with prior known securing devices because there are a limited number of geometric configurations that the known securing devices can be used to both retain an inserted medical device and be affixed to a patient's body. The most favoured configuration for known securing devices is to have the inserted medical device forced to lie in longitudinal contact with the patient's skin. This is not always the most appropriate orientation for the medical device.

## Disclosure of Invention

According to the present invention a securing device for an inserted needle, cannula or the like at an intravenous site comprises: a first element that is adapted to be secured to a patient; and, a second element retained by the first element that is a body of pliable material which is mouldable about a portion of the inserted medical device for securing the medical device in combination with the first element to the patient.

In a preferred arrangement for the present invention the first element can be in the nature of a flexible adhesive strip. Alternatively the first element could be in the nature of a strap adapted to encircle a limb of a patient. In most cases it is preferred that the strip or strap will be transparent.

Further according to the present invention the second element that is a body of pliable material can preferably have an adhesive character. Preferably also the second element of pliable material can be transparent and could have antimicrobial properties.

Transparency of the first and second elements permits visual examination of the site where the medical device enters the patient's body.

The present invention further envisages a securing device that could have a protective peel-off layer for the first element adhesive strip as well as for the second element pliable material.

## Brief Description of the Drawings

The various objects, advantages and novel features of the present invention will become more readily apprehended from the following detailed description when taken in conjunction with the appended drawings, in which:

FIGURE 1 is a schematic perspective view of a securing device in accordance with the present invention in an opened state;

FIGURE 2 is a schematic perspective view of a securing device in accordance with the present invention in a folded state; and

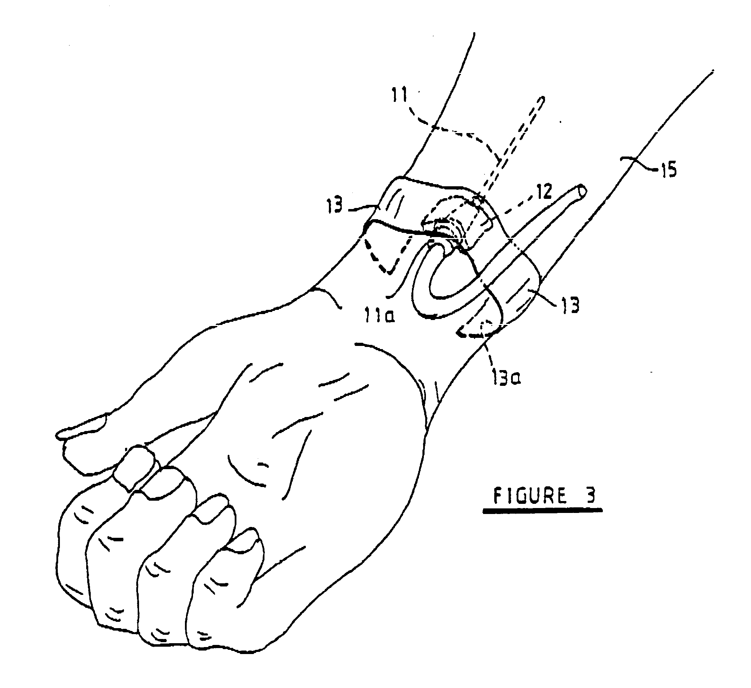

FIGURE 3 is a schematic partially broken away view of the device in Figure 1 in use.

Referring to the drawings a securing device, generally designated by reference numeral 10, in accordance with the present invention is shown. This securing device 10 is adapted for securing an inserted needle or cannula 11 at the site where the medical device enters the patient's body, and it comprises a body of pliable adhesive material 12 moulded to the hub or trailing end 11a of the cannula or needle 11. The pliable material 12 can preferably be a putty-like adhesive substance such as hydrogel with a consistency so as to enable the cannula or needle 11 to be embedded in the material by using gentle finger pressure to mould the necessary shape. A material marketed under the trade name "PRESS STICK" has been found to have a suitable consistency and to also have appropriate adhesive qualities. It is preferred that the pliable material 12 be transparent such as some hydrogels are so as not to obscure the intravenous site, and in addition the material can act as a carrier for antimicrobial agents. The pliable material 12 should be thick enough so that when moulded to the cannula or needle 11 and patient's skin, the moulded pliable material 12 provides trauma and pull out protection. It has been found that at least 100ml of pliable material 12 is advantageous.

The device of the present invention further includes a system for securing the pliable material 12 with the cannula or needle 11 to a patient. For the preferred embodiment, such means comprises a strip 13 of flexible transparent material having an adhesive layer 13a thereon adapted for adhering to the skin of a patient. Other shapes than that shown in the figures for strip 13 can be used. It will be appreciated that as an alternative a limb encircling strap or the like, not shown, could be employed if desirable for securing the pliable material 12.

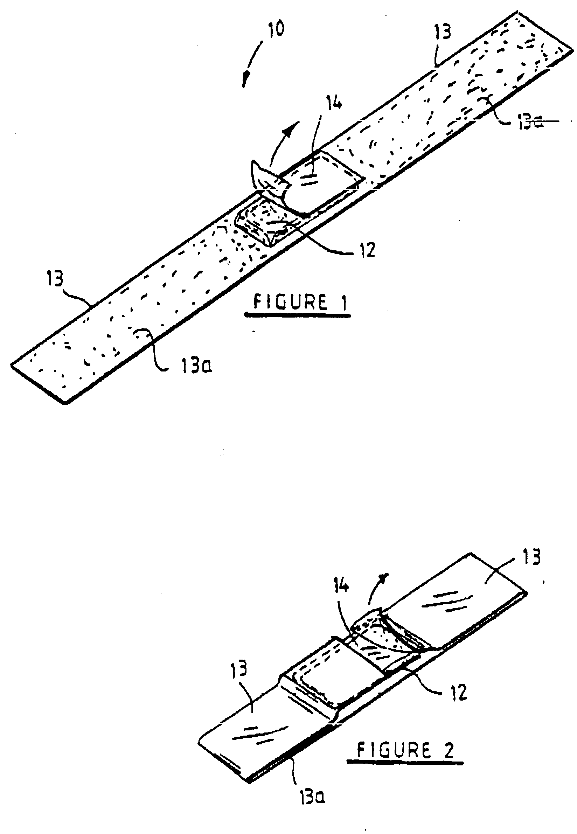

For packaging purposes a protective peel-off strip 14 can preferably be provided at least for the pliable material 12 as illustrated. It is noted that in the arrangement illustrated in Figure 2 the adhesive strip 13 is folded back on itself on both sides to protect the adhesive layer 13a and is designed to be opened up for use. Clearly variations are possible in this regard. For example a protective peel-off strip, not shown, could be provided for the adhesive strip 13.

In use the device of the present invention will be opened up, see Figure 1, so the adhesive 13a of the strip 13 is exposed and the peel-off strip 14 removed to expose the pliable material 12. Then the strip 13 can be secured to a patient's skin 15 transversely relative to a cannula or needle 11, with the pliable material 12 in contact with the hub 11a of the latter. In order to embed the hub 11a in the pliable material 12, gentle finger pressure will be applied to the outer surface or the tape 13 to mould the pliable material 12 around the hub 11a of the cannula 11. Alternatively the peel-off strip 14 can be removed and the pliable material 12 moulded by gentle finger pressure around the hub 11a. Then the adhesive 13a of the strip 13 can be exposed and the securing device 10 can be secured to a patient's skin 15.

The above discussion and related illustrations of the present invention are directed primarily to a preferred embodiment and practices of the invention. However, it is believed that numerous changes and modifications in the actual implementation of the concepts described herein will be apparent to those skilled in the art, and it is contemplated that such changes and modifications may be made without departing from the scope of the invention as defined by the following claims.

## Claims

1. A protecting and securing device for use with a medical needle or cannula, characterized in that the device comprises a first element adapted to be secured to a patient and a second element, retained by the first element and comprising a body of pliable material mouldable about a portion of the medical needle or cannula.

2. A device as claimed in claim 1 wherein the first element comprises a flexible strip, the second element being mounted on one side of the strip.

3. A device as claimed in claim 2 wherein the side of the strip on which the second element is mounted is coated with an adhesive.

4. A device as claimed in any one of claims 1 to 3 wherein the pliable material is adhesive.

5. A device as claimed in claim 3 or claim 4 wherein either or both of the first and second elements is covered with a peel-off backing material.

6. A device as claimed in any one of claims 1 to 5 wherein the pliable material is impregnated with one or more antimicrobial agents.

7. A device as claimed in claim 6 wherein the antimicrobial agent comprises an antibacterial agent.

8. A device as claimed in claim 7 wherein the antibacterial agent comprises an antibiotic agent.

9. A device as claimed in any one of claims 1 to 8 wherein the pliable material is a hydrogel.

10. A device as claimed in any one of claims 1 to 9 wherein at least one of the first and second elements is transparent.

10

14

13

13a

12

13

13a

FIGURE 1

14

13

13

12

13a

FIGURE 2

FIGURE 3

European Patent
Office

**EUROPEAN SEARCH REPORT**

Application Number

EP    91 31 1184

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-2 727 512 (MULLER) <br> * column 2, line 35 - line 45 * <br> * column 3, paragraph 6; claim 1; figures 4,5 * | 1-3,5 | A61M25/02 |
| Y | | 6,7,9,10 | |
| Y | WO-A-8 801 877 (SMITH & NEPHEW ASSOCIATED COMPANIES PLC) <br> * page 7, paragraph 1 * <br> * page 9, line 5 - line 10 * <br> * page 15, paragraph 4 -paragraph 5 * | 6,7,9,10 | |
| A | | 4,8 | |
| X | US-A-4 897 082 (ERSKINE) <br> * abstract * <br> * column 4, last paragraph; figures 1,2 * | 1,2,4,5 | |
| A | DE-A-3 807 944 (B.BRAUN MELSUNGEN AG) <br> * column 3; figures 1,2 * | 1-3,5-7 | |
| A | WO-A-8 901 349 (KALT) <br> * page 9, line 8 - line 21; claim 1; figures 1-4 <br> * | 1-5 | TECHNICAL FIELDS SEARCHED (Int. Cl.5) <br><br> A61M |

The present search report has been drawn up for all claims

| Place of search | Date of completion of the search | Examiner |
|---|---|---|
| BERLIN | 12 MARCH 1992 | ROLAND A. |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons
......................................................................................
& : member of the same patent family, corresponding
 document

EPO FORM 1503 03.82 (P0401)